# EUROPÄISCHE PATENTSCHRIFT

(11) **EP 1 180 381 B1**
(45) Veröffentlichungstag und Bekanntmachung des Hinweises auf die Patenterteilung: **28.12.2005**
(21) Anmeldenummer: 01109231.9
(22) Anmeldetag: 14.04.2001
(51) Int. Cl.: A61M 25/06, A61M 5/32

(54) **Intravenöse Kathetervorrichtung mit Nadelschutz**
Intravenous catheter with needle guard
Cathéter intraveineux muni d'une gaine de protection

(30) Priorität: 14.08.2000 US 638641; 16.03.2001 DE 20104539 U
(43) Veröffentlichungstag der Anmeldung: 20.02.2002
(73) Patentinhaber: B. BRAUN MELSUNGEN AG, 34212 Melsungen (DE)
(72) Erfinder: Woehr, Kevin, 34587 Felsberg (DE)
(74) Vertreter: Klingseisen, Franz

(56) Entgegenhaltungen:
- WO-A-99/08742

## Beschreibung

Die Erfindung betrifft eine intravenöse Kathetervorrichtung nach dem Oberbegriff der Anspruche 1 bis 3.

Eine intravenöse Kathetervorrichtung dieser Art ist bekannt aus WO 99/08 742. Diese Kathetervorrichtung weist einen rohrförmigen Katheter mit am proximalen Ende vorgesehenem Katheteransatz, eine in den Katheter einschiebbare hohle Nadel mit einem am proximalen Ende vorgesehenen Nadelansatz und einen Nadelschutz auf. Der Nadelschutz befindet sich in einem Hohlraum des Katheteransatzes, der von dem Nadelansatz verschlossen wird, wobei die Nadel durch den Nadelschutz hindurch geht und der Nadelschutz aus einem Federteil aus Stahl besteht, durch welches die Nadel hindurchgleiten kann. Der Nadelschutz weist ein Eingriffselement auf, das beim Zurückziehen der Nadel in Bezug auf den Katheter mit einer Irregularität der Nadel zusammengreift, um den Nadelschutz aus dem proximalen Ende des Katheteransatzes herauszuziehen. Der Nadelschutz besteht aus einem gebogenen Blechteil aus Federstahl, welches gegen die Wand des Hohlraums des Katheteransatzes drückt und sich dadurch abstützt. Diese Abstützung endet, wenn beim Zurückziehen der Nadel der Nadelschutz über die Nadelspitze bewegt wird und dadurch entspannt wird. Dann kann der Nadelschutz zusammen mit der Nadel aus dem Hohlraum des Katheteransatzes herausgezogen werden. Bei der bekannten Kathetervorrichtung besteht der Vorsprung mit dem der Nadelschutz rastend zusammengreift aus einer Rückhaltenase, die der Innenwand des Katheteransatzes angeformt ist, oder aus einer Rückhaltenut.

Bei einer Kathetervorrichtung mit Nadelschutz besteht die Schwierigkeit, den Nadelschutz in dem Katheteransatz zu montieren und dabei eine Biegung oder ein Knie des Nadelschutzes über den Vorsprung an der Innenseite des Katheteransatzes hinwegzubringen, so dass ein sicherer Halt des Nadelschutzes in dem Hohlraum des Katheteransatzes gewährleistet ist.

Der vorliegenden Erfindung liegt die Aufgabe zugrunde, eine intravenöse Kathetervorrichtung mit Nadelschutz zu schaffen, bei der die Montage des Nadelschutzes in dem Katheteransatz vereinfacht ist.

Die Lösung dieser Aufgabe erfolgt erfindungsgemäß durch die Merkmale im Kennzeichnenden Teil der Ansprüche 1 bis 3. Nach Anspruch 1 ist der Vorsprung generell ringförmig ausgebildet und er erstreckt sich an mindestens zwei diametralen Stellen des Katheteransatzes. Dies bedeutet, dass der Vorsprung, an dem der Nadelschutz rastend angreift, nicht nur ein örtlich singulärer Vorsprung ist, sondern ein unterbrochener oder geschlossener Ring, der sich generell um den gesamten Umfang des Hohlraums erstreckt. Wie nachfolgend noch erläutert wird, kann der generell ringförmige Vorsprung eine, zwei oder mehr Durchbrechungen, insbesondere vier Durchbrechungen, aufweisen. Dabei erstreckt sich der Vorsprung jeweils über einen Umfangsbereich von mindestens 60° kontinuierlich. Es ist daher nicht erforderlich, den Nadelschutz in einem vorbestimmten Drehwinkel in Bezug auf den Katheteransatz präzise zu montieren. Auch nachträgliche Verdrehungen des Nadelschutzes sind möglich, ohne dass die Funktion beeinträchtigt wird.

Der Vorsprung kann aus einem kontinuierlichen Ring ohne jegliche Unterbrechung bestehen. In diesem Fall muss der Nadelschutz bei der Montage im Bereich der Biegung vorübergehend stark deformiert werden, damit die Biegung den Vorsprung überwinden kann. Bei anderen Varianten, bei denen der Vorsprung mindestens eine Durchbrechung hat, ist eine solche stärkere Deformierung des Nadelschutzes bei der Montage nicht erforderlich.

Die Nadel kann an einer Stelle ihrer Länge so ausgebildet sein, dass sie mit dem Eingriffselement des Nadelschutzes zusammengreift, um den Nadelschutz rotatorisch mit der Nadel zu koppeln. In diesem Fall ist es möglich, den Nadelansatz zu verdrehen und dadurch auch den Nadelschutz im Innern des Hohlraums des Katheteransatzes mitzudrehen. Auf diese Weise kann der Nadelschutz in eine Drehposition gebracht werden, in der er den Vorsprung passieren kann, und in eine andere Drehposition, in der er an dem Vorsprung einrastet. Auf diese Weise ist eine besonders einfache Montage des Nadelschutzes in dem Hohlraum des Katheteransatzes möglich, ohne starke Kräfte einwirken zu lassen.

Im Folgenden werden unter Bezugnahme auf die Zeichnungen Ausführungsbeispiele der Erfindung näher erläutert.

Es zeigen:
- Fig. 1: ein Längsschnitt durch eine intravenöse Kathetervorrichtung nach der Erfindung bei einem ersten Ausführungsbeispiel,
- Fig. 2: das distale Ende der Nadel mit dem die Nadelspitze schützenden Nadelschutz nach dem Herausziehen der Nadel aus dem Katheter,
- Fig. 3: einen Schnitt entlang der Linie III-III von Fig. 1,
- Fig. 4: in ähnlicher Darstellung wie Fig. 3, eine zweite Ausführungsform mit im Wesentlichen ringförmigem Vorsprung, der eine kleine Unterbrechung aufweist,
- Fig. 5: einen Schnitt durch eine dritte Ausführungsform mit einem zweiteiligen ringförmigen Vorsprung, dessen Segmente durch zwei Unterbrechungen getrennt sind,
- Fig. 6: eine schematische Seitenansicht des distalen Bereichs der Nadel von Fig. 1,
- Fig. 7: eine Ansicht der Nadel von Fig. 6, aus Richtung des Pfeiles VII,
- Fig. 8: eine perspektivische Darstellung eines Werkzeugs für die Montage des Nadelschutzes bei einem Ausführungsbeispiel der Erfindung,
- Fig. 9: einen schematischen Querschnitt einer weiteren Ausführungsform der Erfindung, wobei der Nadelschutz so orientiert ist, dass er in den Hohlraum des Katheteransatzes eingeschoben werden kann und
- Fig. 10: von gleicher Darstellung wie Fig. 9, den Zustand des Nadelschutzes nach Drehung um etwa 45°, wobei der Nadelschutz die Segmente des Vorsprungs hintergreift und an dem Vorsprung verrastet ist.

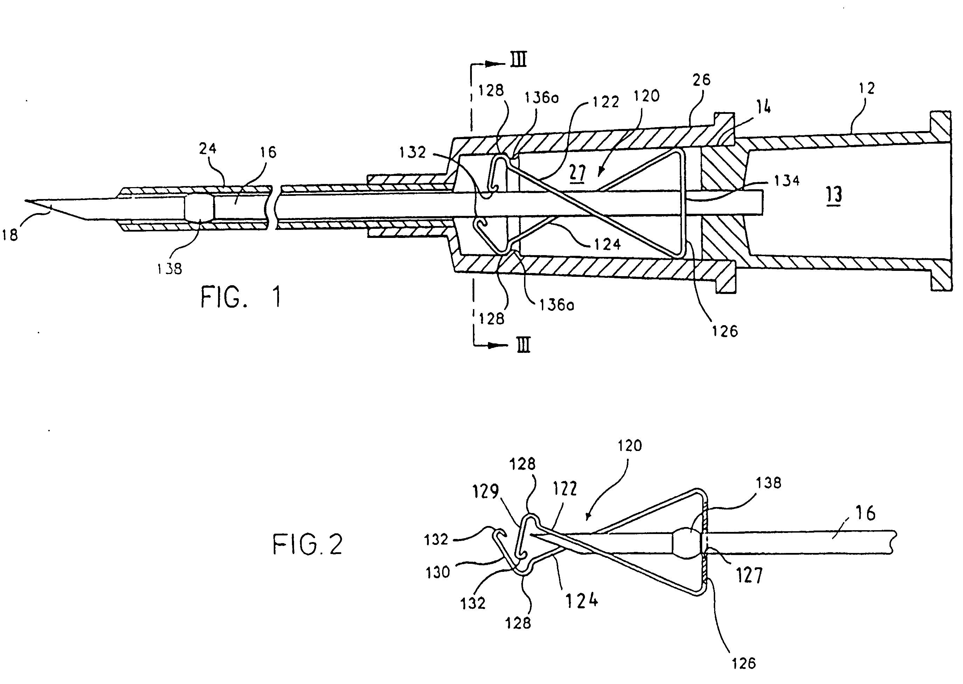

Das in Fig. 1 dargestellte Ausführungsbeispiel der vorliegenden Erfindung weist einen langgestreckten hohlen rohrförmigen Katheter 24 auf, der an seinem (patientenfernen) proximalen Ende konzentrisch mit dem distalen Ende eines Katheteransatzes 26 verbunden ist. Der Katheteransatz 26 enthält einen Hohlraum 27, der mit dem Volumen des Katheters 24 in Verbindung steht und der am proximalen Ende offen ist.

Durch den Katheter 24 verläuft die Nadel 16. Die Nadel 16 ist eine langgestreckte Hohlnadel aus Stahl, die am distalen Ende eine Nadelspitze 18 aufweist. Ein Stück entfernt von dem distalen Ende 18 befindet sich eine Irregularität 138. Die Irregularität 138 besteht bei dem vorliegenden Ausführungsbeispiel aus einem abgeflachten Abschnitt der Nadel, wodurch sich in der einen Richtung die Breite (der Durchmesser) der Nadel vergrößert und in der hier zu senkrechten Richtung verkleinert. Die Irregularität 138 kann alternativ aus einer querlaufenden Kerbe oder Rille an der Außenseite, oder aus einem lokalen Vorsprung bestehen.

An dem proximalen Ende der Nadel 16 ist ein Nadelansatz 12 befestigt, der einen Hohlraum 13 aufweist, welcher mit dem Volumen der Nadel in Verbindung steht. Der Nadelansatz 12 ist becherförmig ausgebildet und er ist am proximalen Ende offen. Am distalen Ende weist der Nadelansatz 12 eine konische Passfläche 14 auf, die in das distale Ende des Katheteransatzes 26 passend eingesetzt werden kann, um den Katheteransatz in definierter Position am Nadelansatz festzulegen. In dieser Relativposition des Nadelansatzes 12 zum Katheteransatz 26 ragt das distale Ende der Nadel 16 mit der Nadelspitze 18 aus dem distalen Ende des Katheters 24 heraus.

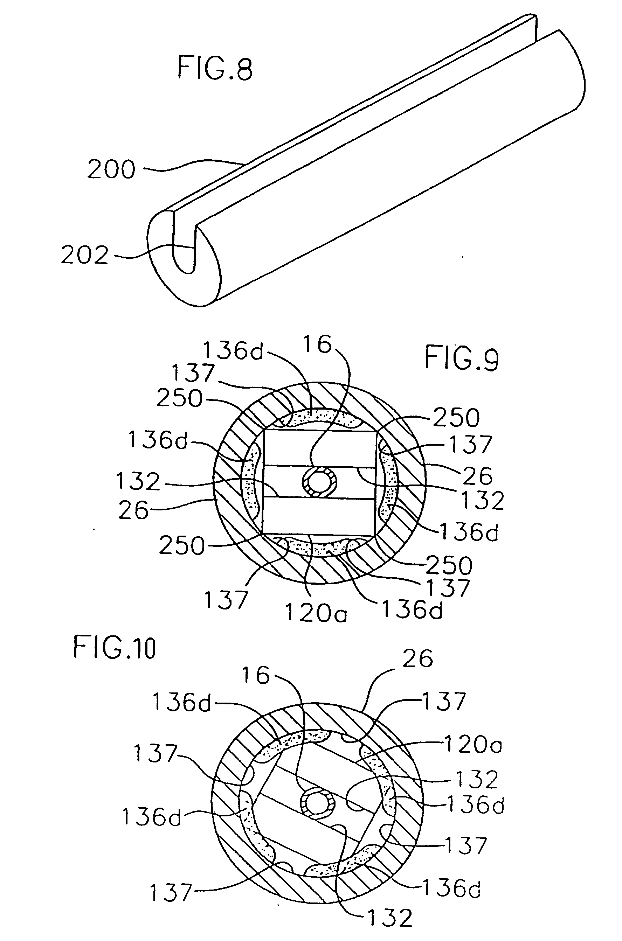

Auf der Nadelspitze 18 sitzt ein Nadelschutz 120, der in generell in gleicher Weise ausgebildet ist wie der Nadelschutz, der in den Fig. 10A, 10B und 11 von WO 99/08 742 dargestellt und beschrieben ist. Der Nadelschutz kann jedoch auch hiervon abweichend gestaltet sein, wofür es im Stand der Technik zahlreiche Anregungen gibt. Insbesondere braucht der Nadelschutz keine Klemmeinrichtungen aufzuweisen, um eine Klemmung an der Nadel zu bewirken.

Der Nadelschutz 120, der im gespannten Zustand in Fig. 1 und im entspannten Zustand in Fig. 2 dargestellt ist, besteht aus einem einstückigen Teil aus Federstahl. Er weist eine quer zur Nadel 16 verlaufende Rückenwand 126 auf, die eine Öffnung enthält. Die Öffnung bildet das Eingriffselement 127, das mit der Irregularität 138 der Nadel 16 zusammengreift. Die Öffnung bzw. das Eingriffselement 127 ist so bemessen, dass sie auf der Nadel 16 frei verschoben werden kann, jedoch die Irregularität 138 nicht passieren kann. Folglich ist die Rückenwand in dem Bereich zwischen der Irregularität 138 und dem Nadelansatz 12 verschiebbar.

Von den Enden der Rückenwand 126 des Nadelschutzes 120 stehen Arme 122 und 124 ab, die generell in distale Richtung weisen und einander überkreuzen, wobei sich jeder Arm 122,124 auf einer anderen Seite der Nadel 16 befindet. Am Ende eines jeden Armes 122,124 befindet sich eine Endwand 129,130, die in Bezug auf den Arm abgewinkelt ist. An dem Übergang zwischen dem Arm und der Endwand befindet sich eine Beigung (128) mit nach außen gerichteter Wölbung, bzw. ein Knie. An dem freien Ende der Stirnwand 129, bzw. 130, ist eine um mehr als 90° umgebogene Lippe 132 vorhanden. Der Arm 122 ist kürzer als der andere Arm 124, so dass die Stirnwände 129,130 im entspannten Zustand des Nadelschutzes nicht miteinander kollidieren. Die Stirnwände 129 und 130 haben eine größere Breite als die Arme 122,124, so dass sie im entspannten Zustand die Nadelspitze 18 bedecken und gegen Berührung mit anderen Teilen oder Körpern schützen können.

Der Nadelschutz 120 wird in dem Katheteransatz 26 festgehalten. Hierzu dient ein ringförmiger Vorsprung 136a, der an der Innenwand des Katheteransatzes 26 umläuft. Während die Lippen 132 von entgegensetzten Seiten her gegen die Außenwand der Nadel 16 drücken (Fig. 1), hintergreifen die Biegungen 128 den Vorsprung 136a. Wird die Nadel 16 aus der Katheter 24 herausgezogen, so wird der Nadelschutz 120 zunächst in dem Katheteransatz 26 festgehalten, so dass die Nadel 16 durch ihn hindurchgleitet. Wenn die Nadelspitze die Lippen 132 passiert, federn die Arme 122,124 in die in Fig. 2 dargestellte Position, in der die Stirnwände 129,130 die Nadelspitze überdecken. In diesem Zustand stößt das Eingriffselement 127 gegen die Irregularität 138, so dass der Nadelschutz 20 daran gehindert wird, über das distale Ende der Nadel 16 herauszugleiten.

Bei dem Ausführungsbeispiel der Fig. 1 ist der Vorsprung 136a vollständig rund und kontinuierlich. Der ringförmige Vorsprung 136a hält den Nadelschutz 120 in dem Katheteransatz 26 fest, wenn die Nadel 16 von dem Katheteransatz 26 abgezogen wird, bis die Arme 122,124 des Nadelschutzes 120 ihrer Abstützung an der Nadel 16 verlieren und dadurch nach innen federn. Der Vorsprung 136 ist hier dem Katheteransatz 26 einstückig angeformt, beispielsweise im Spritzgussverfahren.

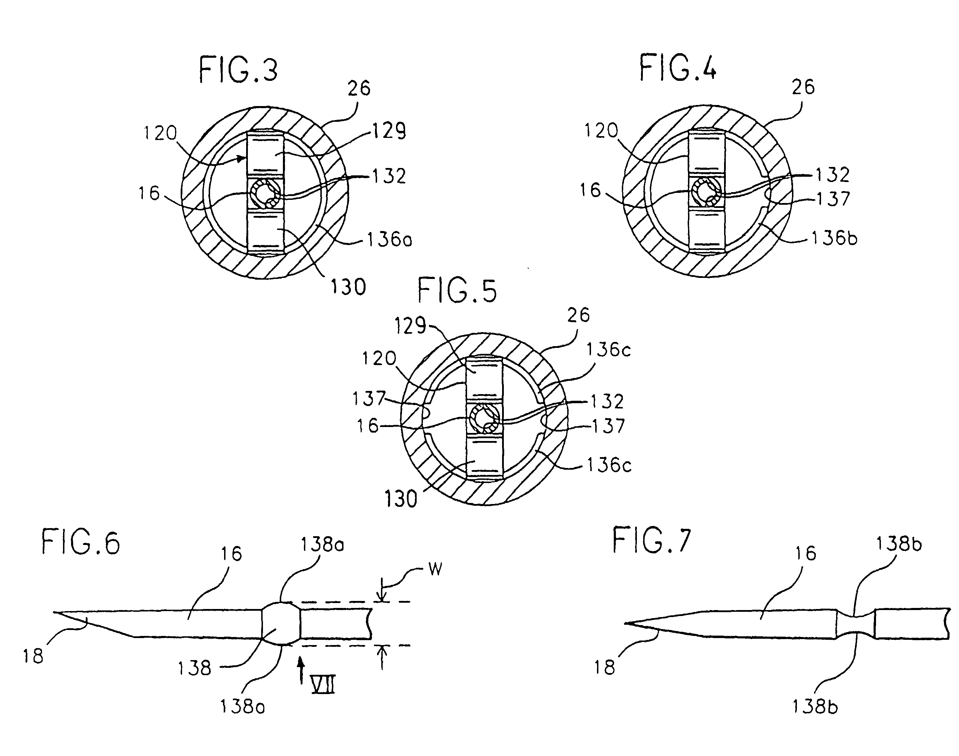

Bei dem Ausführungsbeispiel von Fig. 4 hat der nach innen gerichtete Vorsprung 136b eine generell C-förmige Konfiguration. Der C-förmige Vorsprung 136b kann aus einem metallischen Federring bestehen, der teilweise in einer Ringnut an der Innenseite des Katheteransatzes sitzt. Auf diese Weise besteht die Möglichkeit, den Vorsprung 136b dadurch zu erzeugen, dass der Federring in den Katheteransatz 26 eingeschoben wird, bis er in die entsprechende Nut einschnappt.

Fig. 5 zeigt eine weitere Alternative, bei der der Vorsprung 136c in zwei Segmente unterteilt ist, zwischen denen sich Unterbrechungen 137 befinden. Jedes Segment hat eine größere Umfangserstreckung als die dazwischen befindlichen Unterbrechungen 137. Ferner ist die Umfangserstreckung wesentlich größer als die Breite der distalen Stirnwände 129,130 des Nadelschutzes. Es wird bevorzugt, dass der nach innen vorstehende generell ringförmige Vorsprung keine Unterbrechungen 137 hat, deren Breite größer ist als die Breite des distalen Stirnwände 129 bzw. 130 des Nadelschutzes. Die Bemessung der Unterbrechungen 137 derart, dass sie kleiner sind als die Breite der Stirnwände, verhindert, dass der Nadelschutz 120 unbeabsichtigt die Unterbrechungen 137 passiert und der Nadelschutz 120 dadurch aus dem Katheteransatz 26 herausgleiten kann, ohne die Nadelspitze zu bedecken. Entsprechend der vorliegenden Erfindung kann jede beliebige Zahl separater Segmente vorgesehen sein, die einen generell ringförmigen Vorsprung bilden, solange der Nadelschutz 120 wirksam in dem Katheteransatz festgehalten wird, während die Nadel 16 aus dem Katheter 24 herausgezogen wird.

Wie in den Fig. 6 und 7 dargestellt ist, wird die Irregularität 138 der Nadel 16 vorzugsweise durch eine Quetschung erzeugt, bei der zwei nach entgegengesetzten Richtungen weisende Ausbeulungen 138a entstehen, sowie rechtwinklig hierzu zwei aneinander ebenfalls gegenüberliegende Eindrückungen 138B. Die Ausbeulungen 138a bilden eine Breite W, die klein genug ist, um die Bewegung der Nadel 16 im Katheter 24 gemäß Fig. 1 zu ermöglichen, die jedoch zu groß ist, um die das Eingriffselement 127 bildende Öffnung in der Basiswand 126 des Nadelschutzes 120 passieren zu können.

Die Kathetervorrichtung nach Fig. 1 wird zusammengebaut, indem der Nadelschutz 120 über die Spitze 18 der Nadel 16 geschoben wird, bevor die Irregularität 138 an der Nadel erzeugt wird. Die Nadel 16 passiert dann das Eingriffselement 134, das aus einer Öffnung in der Basiswand 126 des Nadelschutzes 120 besteht. Die distalen Arme 132 werden auseinander gedrückt und die Nadel 16 geht zwischen ihnen hindurch. Dann wird die Irregularität 138 an der Nadel 16 erzeugt, so dass der Nadelschutz 120 zwischen der Irregularität 138 und dem Nadelansatz 12 gefangen ist.

Als nächstes wird die Nadel 16 mit dem darauf befindlichen Nadelschutz 120 in den Katheteransatz 26 eingeschoben, so dass die scharfe Nadelspitze 18 in den Katheter 24 eintritt. Die Nadel 16 wird so weit in den Katheter 24 eingeschoben, bis die Biegungen 128 des Nadelschutzes gegen den Vorsprung 136a stoßen.

Wenn die Biegungen 128 gegen den ringförmigen Vorsprung 136a stoßen, endet die Vorwärtsbewegung des Nadelschutzes. Diese Vorwärtsbewegung kann auch bereits früher enden, weil die Innenwand des Katheteransatzes 26 sich verjüngt, so dass der Nadelschutz 120 mit zunehmendem Vorschieben zusammengedrückt wird. Wenn der Nadelschutz 120 sich nicht vorschieben lässt, wird ein Werkzeug benutzt, um den Nadelschutz 120 weiter in den Katheteransatz 26 vorzuschieben, bis die Biegungen 128 des Nadelschutzes 120 über den Vorsprung 136a schnappen. Hierbei muss eine hinreichende Kraft mit dem Werkzeug auf den Nadelschutz 120 ausgeübt werden, so dass die Biegungen 128 gegeneinander federn, um den Vorsprung 136a zu überwinden. Danach biegen sie sich wieder nach außen zurück und drücken gegen die Innenwand des Katheteransatzes 26. Der Nadelschutz 120 ist nun in der Bereitschaftsposition, die in Fig. 1 dargestellt ist.

Das Werkzeug zum Einschieben des Nadelschutzes kann jede geeignete Form haben. Vorzugsweise wird das in Fig. 8 dargestellte Werkzeug benutzt, das aus einem einfachen Stift 200 mit einem längslaufenden Schlitz 202 besteht, der sich über die gesamte Länge erstreckt. Das Werkzeug 200 kann in dem Schlitz 202 die Nadel 16 aufnehmen, während es den Nadelschutz 120 vorschiebt.

Der ringförmige Vorsprung 136a hält den Nadelschutz 120 beim Zurückziehen der Nadel fest, bis die Lippen 132 des Nadelschutzes 120 über die Nadelspitze schnappen, so dass die Biegungen 128 von dem ringförmigen Vorsprung 136a freikommen und der Nadelschutz nunmehr zusammen mit der Nadel 16 aus dem Katheteransatz 26 herausgezogen werden kann.

Eine alternative Methode zum Zusammenbau der intravenösen Kathetervorrichtung mit Federklemme nach der vorliegenden Erfindung ergibt sich bei Benutzung der in Fig. 4 dargestellten Ausführungsform, bei der der Vorsprung 136b aus einem offenen Federring besteht. In diesem Fall kann der offene Federring auf den Nadelschutz aufgeschoben werden, so dass er sich zwischen dessen proximalem und distalem Ende befindet. Zuvor wurde der Nadelschutz 120 auf die Nadel 16 aufgeschoben und diese dann durch Herstellung der Irregularität 138 deformiert. Dann werden der Nadelschutz 120 und der offene Ring 136b gleichzeitig, unter Verwendung desselben Werkzeugs, in den Katheteransatz 26 eingeschoben.

Dieses Werkzeug hat vorzugsweise zwei Finger, von denen jeder sich entlang einer Seite des Nadelschutzes 120 erstreckt. Mit diesen Fingern wird der offene Ring 136b weiter in den Katheteransatz hineingeschoben als das proximale Ende des Nadelschutzes 120. Auf diese Weise ist es nicht erforderlich, den Nadelschutz 120 über den ringförmigen Vorsprung 136b zu schieben. Dagegen werden Nadelschutz 120 und ringförmiger Vorsprung 136b gemeinsam eingesetzt. Der ringförmige Vorsprung 136b wird daher derart in den Katheteransatz 26 eingefügt, dass der Nadelschutz 120 durch ihn verrastet wird und solange im verrasteten Zustand bleibt, bis die Nadel 16 zurückgezogen wird.

In den Fign. 9 und 10 ist eine weitere Ausführungsform der Vorrichtung dargestellt, die eine andere Montageart ermöglicht. Hierbei ist der ringförmige Vorsprung 136d so ausgebildet, dass er mindestens 4 Durchbrechungen 137 zwischen den Segmenten aufweist, wobei die Ecken 250 des Nadelschutzes 120a mit geringer oder keiner Kraft durch die Durchbrechungen 137 geschoben werden können. Nachdem der Nadelschutz 120a bis hinter den Vorsprung 136d in den Katheteransatz 26 eingeschoben wurde, wird der Nadelschutz 120a gedreht, so dass seine vier Ecken 250 hinter den Segmenten des Vorsprungs 136d zu liegen kommen, so wie dies in Fig. 10 dargestellt ist. Dadurch wird der Nadelschutz 120a mit dem Katheteransatz 26 festgehalten, bis die Nadel 16 herausgezogen wird.

Es kann zweckmäßig sein, den Nadelschutz 120 so auszubilden, dass er ein generell quadratisches Profil hat, wie dies in den Fig. 9 und 10 dargestellt ist, jedoch können auch andere rechteckige oder mehrkantige Konturen für den Nadelschutz 120 A benutzt werden. Es ist lediglich erforderlich, die Durchbrechungen 137 des Vorsprungs 136d so anzuordnen, dass der Nadelschutz 120 während der Montage leicht hindurchgeht und anschließend nach einer Drehung des Nadelschutzes 120a verrastet wird, so dass die Ecken von 250 gegen die Segmente des Vorsprungs 136d stoßen.

## Patentansprüche

1. Intravenöse Kathetervorrichtung mit einem rohrförmigen Katheter (24), der ein proximales Ende mit einem Katheteransatz (26) aufweist, einer in den Katheter (24) einschiebbaren Nadel (16), die ein proximales Ende mit einem Nadelansatz (12) aufweist, wobei der Nadelansatz (12) derart mit dem Katheteransatz (26) zusammengreift, dass eine Spitze (18) am distalen Ende der Nadel (16) aus dem Katheter (24) vorsteht, einem auf der Nadel (16) verschiebbaren einstückigen Nadelschutz (120), der in einem Hohlraum (27) des Katheteransatzes (26) angeordnet ist und ein Eingriffselement (127) aufweist, das beim Zurückziehen der Nadel (16) in Bezug auf den Katheter (24) mit einer Irregularität (138) der Nadel (16) zusammengreift, durch die zwei nach entgegengesetzten Richtungen weisende Ausbeulungen (138a) entstehen sowie rechtwinklig hierzu zwei einander ebenfalls gegenüber liegende Eindrückungen (138b), um den Nadelschutz (120) aus dem proximalen Ende des Katheteransatzes (26) herauszuziehen, wobei der Nadelschutz (120) an der Innenwand des Katheteransatzes anliegende Biegungen (128) an einander gegenüberliegenden Armen (122, 124) sowie Lippen (132) an den freien Enden der Arme aufweist, die von entgegengesetzten Seiten her gegen die Außenwand der Nadel (16) drücken,
**dadurch** gekenntzeichnet,
dass in dem Hohlraum (27) des Katheteransatzes (26) ein Vorsprung (136a, 136b, 136c, 136d) vorsteht, der ein Rückhalteelement für den Nadelschutz (120) bildet, generell ringförmig ausgebildet ist und sich an mindestens zwei diametralen Stellen des Katheteransatzes (26) erstreckt, wobei beim Einschieben des Nadelschutzes (120) in den Katheteransatz die Biegungen (128) der Arme federnd durch den Vorsprung zusammengedrückt werden und dann den Vorsprung hintergreifen, um den Nadelschutz im Katheteransatz zu halten, während die Lippen (132) gegen die Nadelwand drücken, und wobei beim Herausziehen der Nadel die Nadelspitze die Lippen (132) passiert, sodass die Lippen (132) federnd nach innen schwenken und die Nadelspitze überdekken, während die Biegungen (12) am Vorsprung freigegeben werden und der Nadelschutz aus dem Katheteransatz zusammen mit der Nadel herausgezogen werden kann.

2. Intravenöse Kathetervorrichtung mit einem rohrförmigen Katheter (24), der ein proximales Ende mit einem Katheteransatz (26) aufweist, einer in den Katheter (24) einschiebbaren Nadel (16), die ein proximales Ende mit einem Nadelansatz (12) aufweist, wobei der Nadelansatz (12) derart mit dem Katheteransatz (26) zusammengreift, dass eine Spitze (18) am distalen Ende der Nadel (16) aus dem Katheter (24) vorsteht, einem auf der Nadel (16) verschiebbaren einstückigen Nadelschutz (120), der in einem Hohlraum (27) des Katheteransatzes (26) angeordnet ist und ein Eingriffselement (127) aufweist, das beim Zurückziehen der Nadel (16) in Bezug auf den Katheter (24) mit einer Irregularität (138) der Nadel (16) zusammengreift, durch die zwei nach entgegengesetzten Richtungen weisende Ausbeulungen (138a) entstehen sowie rechtwinklig hierzu zwei einander ebenfalls gegenüber liegende Eindrückungen (138b), um den Nadelschutz (120) aus dem proximalen Ende des Katheteransatzes (26) herauszuziehen, wobei der Nadelschutz (120) an der Innenwand des Katheteransatzes anliegende Biegungen (128) an einander gegenüberliegenden Armen (122, 124) sowie Lippen (132) an den freien Enden der Arme aufweist, die von entgegengesetzten Seiten her gegen die Außenwand der Nadel (16) drücken,
**dadurch gekennzeichnet, daß** in dem Hohlraum (27) des Katheteransatzes (26) ein Vorsprung (136d) vorsteht, welcher aus vier Segmenten besteht, die durch vier Unterbrechungen (137) getrennt sind, wobei der Nadelschutz (120) - in axialer Richtung zur Nadel gesehen - vier Ecken (250) an den Biegungen (128) aufweist, die durch die Unterbrechungen (137) hindurchpassen, sodass der Nadelschutz (120a) bis hinter den Vorsprung (136d) in den Katheteransatz 26 eingeschoben und gedreht werden kann, sodass seine vier Ecken (250) hinter den Segmenten dieses Vorsprungs (136d) zu liegen kommen, um den Nadelschutz im Katheteransatz zu halten.

3. Intravenöse Kathetervorrichtung mit einem rohrförmigen Katheter (24), der ein proximales Ende mit einem Katheteransatz (26) aufweist, einer in den Katheter (24) einschiebbaren Nadel (16), die ein proximales Ende mit einem Nadelansatz (12) aufweist, wobei der Nadelansatz (12) derart mit dem Katheteransatz (26) zusammengreift, dass eine Spitze (18) am distalen Ende der Nadel (16) aus dem Katheter (24) vorsteht, einem auf der Nadel (16) verschiebbaren einstückigen Nadelschutz (120), der in einem Hohlraum (27) des Katheteransatzes (26) angeordnet ist und ein Eingriffselement (127) aufweist, das beim Zurückziehen der Nadel (16) in Bezug auf den Katheter (24) mit einer Irregularität (138) der Nadel (16) zusammengreift, durch die zwei nach entgegengesetzten Richtungen weisende Ausbeulungen (138a) entstehen sowie rechtwinklig hierzu zwei einander ebenfalls gegenüber liegende Eindrückungen (138b), um den Nadelschutz (120) aus dem proximalen Ende des Katheteransatzes (26) herauszuziehen, wobei der Nadelschutz (120) an der Innenwand des Katheteransatzes anliegende Biegungen (128) an einander gegenüberliegenden Armen (122, 124) sowie Lippen (132) an den freien Enden der Arme aufweist, die von entgegengesetzten Seiten her gegen die Außenwand der Nadel (16) drücken,
**dadurch gekennzeichnet, daß** in dem Hohlraum (27) des Katheteransatzes (26) ein Vorsprung (136b) vorsteht, welcher ein Federring ist, der eine Öffnung (137) aufweist und in eine Nut an der Innenwand des Katheteransatzes (26) eingreift,
wobei der offene Federring (136b) vor dem Einführen des Nadelschutzes in den Katheteransatz auf den Nadelschutz (120) aufschiebbar ist, sodass er sich zwischen dessen proximalem und distalem Ende befindet, worauf der Nadelschutz (120) und der fene Federring gleichzeitig in den Katheteransatz (26) eingeschoben werden, bis der Federring in die Nut an der Innenwand des Katheteransatzes eingreift.

## Claims

1. Intravenous catheter with a tubular catheter (24) having a proximal end with a catheter hub (26), a needle (16) which is insertable in the catheter (24) and has a proximal end having a needle hub (12), wherein the needle hub (12) engages with the catheter hub (26) such that a tip (18) at the distal end of the needle (16) protrudes from the catheter (24), an integral needle guard (120) displaceable on the needle (16) and arranged in a hollow space (27) in the catheter hub (26), and an engaging element (127) which, on withdrawal of the needle (16) in relation to the catheter (24), engages with an irregularity (138) of the needle (16), through which there result two protuberances (138a) pointing in opposite directions and, perpendicular hereto, two depressions (138b) which are also positioned opposite one another, for removal of the needle guard (120) from the proximal end of the catheter hub (26), wherein on arms (122, 124) positioned opposite one another the needle guard (120) has bends (128) abutting on the inner wall of the catheter hub, and on the free ends of the arms the needle guard (120) has lips (132) which press against the outer wall of the needle (16) from opposite sides;
**characterised in that**
in the hollow space (27) of the catheter hub (26), there protrudes a projection (136a, 136b, 136c, 136d) which forms a retaining element for the needle guard (120), is generally annular in shape, and extends at not less than two diametrical positions of the catheter hub (26), wherein when inserting the needle guard (120) in the catheter hub, the bends (128) of the arms are elastically compressed by the projection and then engage the projection for holding the needle guard in the catheter hub, while the lips (132) press against the needle wall, and wherein on removal of the needle, the needle tip passes the lips (132) such that the lips (132) elastically slew inwards and cover the needle tip, while the bends (12) at the projection are released and the needle guard can be removed from the catheter hub together with the needle.

2. Intravenous catheter with a tubular catheter (24) having a proximal end with a catheter hub (26), a needle (16) which is insertable in the catheter (24) and has a proximal end having a needle hub (12), wherein the needle hub (12) engages with the catheter hub (26) such that a tip (18) at the distal end of the needle (16) protrudes from the catheter (24), an integral needle guard (120) displaceable on the needle (16) and arranged in a hollow space (27) in the catheter hub (26), and an engaging element (127) which, on withdrawal of the needle (16) in relation to the catheter (24), engages with an irregularity (138) of the needle (16), through which there result two protuberances (138a) pointing in opposite directions and, perpendicular hereto, two depressions (138b) which are also positioned opposite one another, for removal of the needle guard (120) from the proximal end of the catheter hub (26), wherein on arms (122, 124) positioned opposite one another the needle guard (120) has bends (128) abutting on the inner wall of the catheter hub, and on the free ends of the arms the needle guard (120) has lips (132) which press against the outer wall of the needle (16) from opposite sides;
**characterised in that**
in the hollow space (27) of the catheter hub (26), there protrudes a projection (136d) which consists of four segments separated by four interruptions (137), wherein the needle guard (120), seen in the axial direction in relation to the needle, has four corners (250) at the bends (128) which pass through the interruptions (137) such that the needle guard (120a) can be inserted and rotated in the catheter hub (26) up to and behind the projection (136d), such that its four corners (250) come to lie behind the segments of this projection (136d) for holding the needle guard in the catheter hub.

3. Intravenous catheter with a tubular catheter (24) having a proximal end with a catheter hub (26), a needle (16) which is insertable in the catheter (24) and has a proximal end having a needle hub (12), wherein the needle hub (12) engages with the catheter hub (26) such that a tip (18) at the distal end of the needle (16) protrudes from the catheter (24), an integral needle guard (120) displaceable on the needle (16) and arranged in a hollow space (27) in the catheter hub (26), and an engaging element (127) which, on withdrawal of the needle (16) in relation to the catheter (24), engages with an irregularity (138) of the needle (16), through which there result two protuberances (138a) pointing in opposite directions and, perpendicular hereto, two depressions (138b) which are also positioned opposite one another, for removal of the needle guard (120) from the proximal end of the catheter hub (26), wherein on arms (122, 124) positioned opposite one another the needle guard (120) has bends (128) abutting on the inner wall of the catheter hub, and on the free ends of the arms the needle guard (120) has lips (132) which press against the outer wall of the needle (16) from opposite sides;
**characterised in that**
in the hollow space (27) of the catheter hub (26), there protrudes a projection (136d) which is a spring ring having a hole (137) and engaging in a groove at the inner wall of the catheter hub (26),
wherein before inserting the needle guard in the catheter hub, the open spring ring (136b) can be displaced onto the needle guard (120) such that it is positioned between the proximal and distal ends thereof, whereupon the needle guard (120) and the open spring ring are simultaneously inserted in the catheter hub (26) until the spring ring engages in the groove on the inner wall of the catheter hub.

## Revendications

1. Dispositif de cathéter intraveineux doté d'un cathéter tubulaire (24) comprenant une extrémité proximale avec un porte-cathéter (26), d'une aiguille (16) pouvant être introduite dans le cathéter (24), laquelle aiguille comprenant une extrémité proximale dotée d'un porte-aiguille (12), le porte-aiguille (12) se mettant en prise avec le porte-cathéter (26) de telle sorte qu'une pointe (18) sur l'extrémité distale de l'aiguille (16) fait saillie du cathéter (24), d'une protection d'aiguille (120) en une seule pièce mobile sur l'aiguille (16), laquelle protection étant disposée dans une cavité (27) du porte-cathéter (26) et comprenant un élément de mise en prise (127), qui, au retrait de l'aiguille (16) par rapport au cathéter (24), se met en prise avec une irrégularité (138) de l'aiguille (16), par laquelle deux renflements (138a) orientés dans des directions opposées sont formés ainsi que deux empreintes (138b) également situées à l'opposé l'une de l'autre, perpendiculairement aux renflements, afin d'extraire la protection d'aiguille (120) de l'extrémité proximale du porte-cathéter (26), la protection d'aiguille (120) comprenant des courbures (128) adjacentes à la paroi intérieure du porte-cathéter sur des bras (122, 124) opposés les uns aux autres ainsi que des lèvres (132) sur les extrémités libres des bras, lesquelles lèvres pressent depuis les côtés opposés contre la paroi extérieure de l'aiguille (16),
**caractérisé en ce que**
une saillie (136a, 136b, 136c, 136d) dépasse dans la cavité (27) du porte-cathéter (26), laquelle saillie forme un élément de retenue pour la protection d'aiguille (120); présente généralement une conception annulaire et s'étend sur au moins deux points diamétralement opposés du porte-cathéter (26), sachant qu'à l'introduction de la protection d'aiguille (120) dans le porte-cathéter, les courbures (128) des bras sont comprimées de manière élastique par la saillie et se mettent ensuite en prise avec l'arrière de la saillie, afin de maintenir la protection d'aiguille dans le porte-cathéter, tandis que les lèvres (132) pressent contre la paroi d'aiguille, et, à l'extraction de l'aiguille, la pointe d'aiguille passent par les lèvres (132), de sorte que les lèvres (132) pivotent de manière élastique vers l'intérieur et recouvrent la pointe d'aiguille, tandis que les courbures (12) sur la saillie sont libérées et que la protection d'aiguille peut être extraite du porte-cathéter conjointement avec l'aiguille.

2. Dispositif de cathéter intraveineux doté d'un cathéter tubulaire (24), comprenant une extrémité proximale avec un porte-cathéter (26), d'une aiguille (16) pouvant être introduite dans le cathéter (24), laquelle aiguille comprenant une extrémité proximale dotée d'un porte-aiguille (12), le porte-aiguille (12) se mettant en prise avec le porte-cathéter (26) de telle sorte qu'une pointe (18) sur l'extrémité distale de l'aiguille (16) dépasse du cathéter (24), d'une protection d'aiguille (120) en une seule pièce mobile sur l'aiguille (16), laquelle protection est disposée dans une cavité (27) du porte-cathéter (26) et comprend un élément de mise en prise (127), qui, au retrait de l'aiguille (16) par rapport au cathéter (24), se met en prise avec une irrégularité (138) de l'aiguille (16), par laquelle deux renflements (138a) orientés dans des directions opposées sont formés ainsi que deux empreintes (138b) également situées à l'opposé l'une de l'autre, perpendiculairement aux renflements, afin d'extraire la protection d'aiguille (120) de l'extrémité proximale du porte-cathéter (26), la protection d'aiguille (120) comprenant des courbures (128) adjacentes à la paroi intérieure du porte-cathéter sur des bras (122, 124) opposés les uns aux autres ainsi que des lèvres (132) sur les extrémités libres des bras, lesquelles lèvres pressent depuis les côtés opposés contre la paroi extérieure de l'aiguille (16),
**caractérisé en ce que**
une saillie (136d) dépasse dans la cavité (27) du porte-cathéter (26), laquelle saillie est constituée de quatre segments qui sont séparés par quatre interruptions (137), la protection d'aiguille (120) -vue dans la direction axiale par rapport à l'aiguille-comprenant quatre coins (250) sur les courbures (128), qui traversent les interruptions (137), de sorte que la protection d'aiguille (120a) peut être introduite jusque derrière la saillie (136d) dans le porte-cathéter (26) et être tournée, de sorte que ses quatre coins (250) viennent se situer derrière les segments de cette saillie (136d) afin de maintenir la protection d'aiguille dans le porte-cathéter.

3. Dispositif de cathéter intraveineux doté d'un cathéter tubulaire (24), comprenant une extrémité proximale avec un porte-cathéter (26), d'une aiguille (16) pouvant être introduite dans le cathéter (24), laquelle aiguille comprend une extrémité proximale dotée d'un porte-aiguille (12), le porte-aiguille (12) se mettant en prise avec le porte-cathéter (26) de telle sorte qu'une pointe (18) sur l'extrémité distale de l'aiguille (16) dépasse du cathéter (24), d'une protection d'aiguille (120) en une seule pièce mobile sur l'aiguille (16), laquelle protection est disposée dans une cavité (27) du porte-cathéter (26) et comprend un élément de mise en prise (127), qui, au retrait de l'aiguille (16) par rapport au cathéter (24), se met en prise avec une irrégularité (138) de l'aiguille (16), par laquelle deux renflements (138a) orientés dans des directions opposées sont formés ainsi que deux empreintes (138b) également situées à l'opposé l'une de l'autre, perpendiculairement aux renflements, afin d'extraire la protection d'aiguille (120) de l'extrémité proximale du porte-cathéter (26), la protection d'aiguille (120) comprenant des courbures (128) adjacentes à la paroi intérieure du porte-cathéter sur des bras (122, 124) opposés les uns aux autres ainsi que des lèvres (132) sur les extrémités libres des bras, lesquelles lèvres pressent depuis les côtés opposés contre la paroi extérieure de l'aiguille (16),
**caractérisé en ce que**
une saillie (136b) dépasse dans la cavité (27) du porte-cathéter (26), laquelle saillie est une rondelle élastique, qui comprend une ouverture (137) et se met en prise dans une gorge sur la paroi intérieure du porte-cathéter (26),
la rondelle élastique ouverte (136b), avant d'introduire la protection d'aiguille dans le porte-cathéter, pouvant être glissée sur la protection d'aiguille (120), de sorte qu'elle se situe entre les extrémités proximale et distale de ladite protection, sur quoi la protection d'aiguille (120) et la rondelle élastique ouverte sont introduites simultanément dans le porte-cathéter (26) jusqu'à ce que l'anneau élastique se mette en prise dans la gorge sur la paroi intérieure du porte-cathéter.
